Europäisches Patentamt

European Patent Office

Office européen des brevets

(11) Publication number: **0 434 258 A2**

# EUROPEAN PATENT APPLICATION

(21) Application number: 90313199.3

(22) Date of filing: 05.12.90

(51) Int. Cl.⁵: **A61F 13/02**

(30) Priority: **21.12.89 GB 8928927**

(43) Date of publication of application:
**26.06.91 Bulletin 91/26**

(84) Designated Contracting States:
**DE DK FR**

(71) Applicant: **Smiths Industries Public Limited Company**
**765, Finchley Road**
**London, NW11 8DS(GB)**

(72) Inventor: **Cross, David Edward**
**Hedge End, Hurst Road**
**Rustington, West Sussex(GB)**

(74) Representative: **Flint, Jonathan McNeill**
**SMITHS INDUSTRIES PUBLIC LIMITED**
**COMPANY 765 Finchley Road**
**London NW11 8DS(GB)**

(54) **Adhesive dressing assemblies and methods of dressing.**

(57) An adhesive dressing 2 of a thin, vapour-permeable polyurethane film has a pressure-sensitive adhesive surface 11 protected, prior to use, by two siliconized paper release sheets 2 and 3. The release sheets are folded in half with the lower half 20, 30 adhering to the dressing and the upper half 21, 31 projecting a short distance beyond the edge of the dressing. The folded edges 22 and 32 of the release sheets overlap in the centre of the dressing. The release sheets are less flexible than the dressing so that they maintain the dressing wrinkle-free during handling. The release sheets can be removed when the dressing is held up to the patient's skin by pulling the free ends 23 and 33 of each sheet outwardly.

Fig. 1.

EP 0 434 258 A2

## ADHESIVE DRESSING ASSEMBLIES AND METHODS OF DRESSING

This invention relates to adhesive dressing assemblies comprising a flexible dressing with an adhesive surface for applying to a patient and two release sheets.

Adhesive dressings are usually protected by a release sheet of a waxed or siliconized paper or the like. The release sheet is easily peeled off the article before use, so as to expose the adhesive, which can then be brought into contact with the skin of the patient. The release sheet either extends completely across the adhesive surface or it may be divided into two separate sheets. In either case, at least one of release sheets must removed before the dressing is brought into contact with the skin and thus brings with it certain disadvantages. Firstly, it can be difficult to apply the dressing without wrinkling. Secondly, the dressing has to be positioned correctly initially, otherwise the adhesive will be contaiminated and reduced in effectiveness if it has to be removed and repositioned. Furthermore, after removal of the release sheet, the dressing is handled by gripping between a finger and thumb, one of which contacts the adhesive surface. Any grease, dust or dirt on the finger or thumb which contacts the adhesive will reduce the effectiveness of the adhesive in the contact region. The adhesive on the dressing will form a bond with the finger or thumb which can be difficult to overcome and, in doing so, this may stretch or otherwise distort the dressing causing it to wrinkle. There may also be a transfer of adhesive to the finger or thumb which is messy and can be difficult to remove. Examples of previous dressings are shown US 1,949,271, US 2,233,209, GB 2,035,096A and GB 2,128,479A.

In the Applicants' copending application GB 2226761A they describe a release sheet for use in securing adhesive ostomy bags to a patient. It has been discovered that a similar release sheet can be used to great advantage for bandaging patients. The term 'dressing' is used here, however, to exclude the attachment of ostomy bags or similar collection bags to a patient but includes bandages and drapes.

Where the dressing is in the form of a thin sheet or membrane, it can be very difficult to handle the membrane after removal of the release sheet because of the tendency of the membrane to crease and wrinkle. Such a sheet used for dressing is described in US Reissue patent 31887. An attempt to overcome the difficulties of such materials is described in US 4,372,303 in which a frame is attached to the membrane on the opposite side from the release sheet, so that the frame supports the membrane, after removal of the release sheet.

The frame is removed after the membrane has been adhered to the skin. This arrangement requires the use of additional components which in themselves are adhesive and thereby makes manufacture and disposal more difficult.

It is an object of the present invention to provide an adhesive dressing assembly and its method of use by which these difficulties can be alleviated.

According to one aspect of the present invention there is provided an adhesive dressing assembly of the above-specified kind, characterised in that each release sheet is folded substantially in half, that each sheet has a first half releasably adhered to a respective different part of the dressing and a second half folded back to overlay the first half, that the folded edges of the two release sheets lie adjacent one another, and that the second half of each sheet extends to respective opposite edges of the assembly so that the release sheets can be removed from the dressing when the assembly is held up to the skin by pulling the respective second halves outwardly from opposite edges of the assembly substantially parallel to the dressing so that the release sheets peel away from the adhesive surface from the centre of the dressing.

The folded edge of one release sheet preferably overlaps the folded edge of the other release sheet. The second half of each release sheet may have a free end that projects beyond the edge of the dressing. The release sheets may be less flexible than the dressing, the release sheets maintaining the dressing in a generally flat configuration and controlling wrinkling during handling and application of the dressing to the patient whereby the dressing resumes its flexible nature after removal of the release sheets and application to the patient so as not to interfere with the flexible functioning of the dressing on the patient's skin.

The dressing may be less than 300 micron thick and is preferably less than 100 micron thick. The dressing may comprise a continuous backing layer of an unreinforced polyurethane film and an adhesive surface of a continuous pressure-sensitive adhesive, both the backing layer and the adhesive being permeable to moisture vapour and at least the backing layer being non-permeable to liquid water. The release sheets may be of siliconized paper.

An adhesive dressing assembly and its method of use, in accordance with the present invention, will now be described, by way of example, with reference to the accompanying drawings, in which:

Figure 1

is a perspective view of the assembly before use;

Figures 2 and 3
are side elevation views of the assembly at different stages of use;

Figure 4
is a perspective view of the assembly during use.

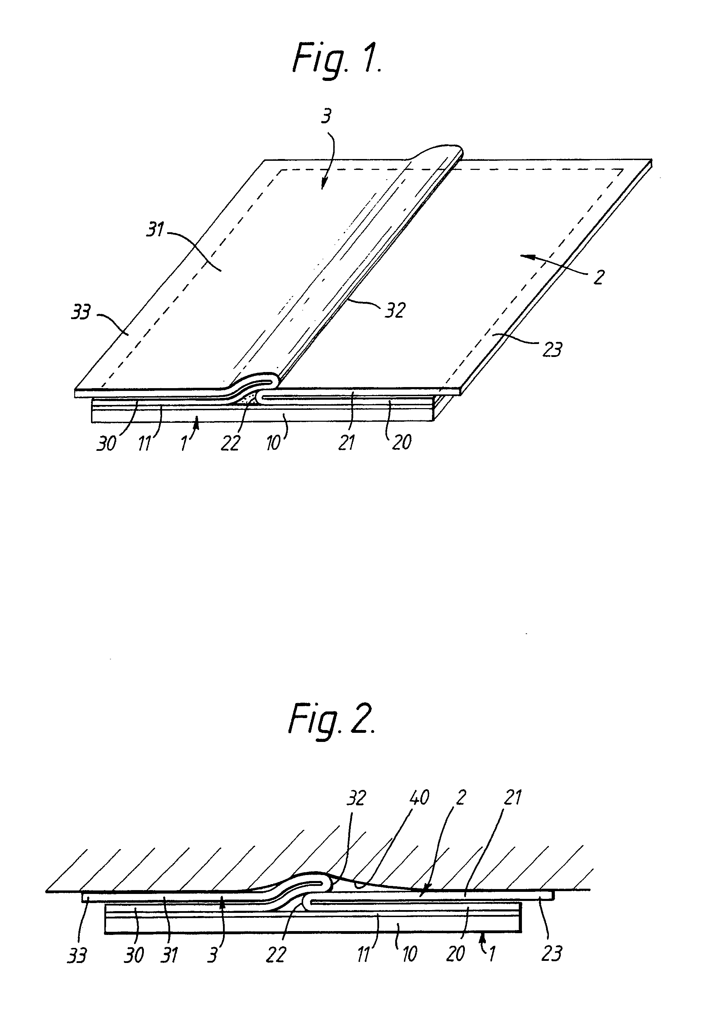

With reference to Figure 1, the adhesive dressing assembly comprises an adhesive flexible dressing 1 and two release sheets 2 and 3.

The dressing 1 is a film which is permeable to oxygen and water vapour but is impermeable to liquid and bacteria; it is highly flexible and thin, typically being less than about 300 micron and preferably less than about 100 micron. The drawings do not show the thickness of the dressing to scale. The dressing 1 is highly stretchable, being capable of stretching to the same extent as the skin without exerting uncomfortable tugging force on the skin. In this respect, the material used to form the dressing is conventional and may be of the kind sold by Smith & Nephew under the name "Op-Site", by 3M under the name 'Tegraderm', by Johnson & Johnson under the name 'Bioclusive', by Hollister under the name 'Thinfilm' or by Howmedica under the name 'Uniflex'. Further details of the material are given in US patents 3645835 (Now Reissue 31,887), 4,372,303 and 4,681,574. Briefly, the dressing 1 comprises a backing layer 10 of a water-vapour-permeable thermoplastic, such as unreinforced polyurethane or similar polymer, with a layer 11 of a pressure-sensitive adhesive on the side which, in use, contacts the skin of the patient. The dressing 1 is illustrated as being rectangular in shape but it will be appreciated that it could be circular or of any other shape.

The release sheets 2 and 3 are of siliconized paper, the thickness of which is such that the release sheets are less flexible than the dressing 1 itself, so that the major structural support for the assembly is provided by the release sheets. Each release sheet 2 and 3 is slightly longer than the dressing and is folded back on itself close to a point midway along its length. The lower half 20 and 30 respectively of the sheets 2 and 3 contacts, and is releasably adhered to, the adhesive surface 11 of the dressing. The second, upper half 21 and 31 of each sheet overlies the first, lower half, with its folded edge 22 and 32 being located towards the centre of the dressing. In this respect, the length of the upper parts 21 and 31 is at least as long as the lower parts 20 and 30, and is preferably slightly longer so that the free ends 22 and 23 of the upper parts project slightly beyond the edge of the dressing. The length of the lower part 20, 30 of each sheet 2 and 3 is slightly longer than half the length of the dressing, so that the folded edge 32 of one sheet 3 overlaps that of the other sheet 2. This ensures that the entire adhesive surface of the dressing is protected.

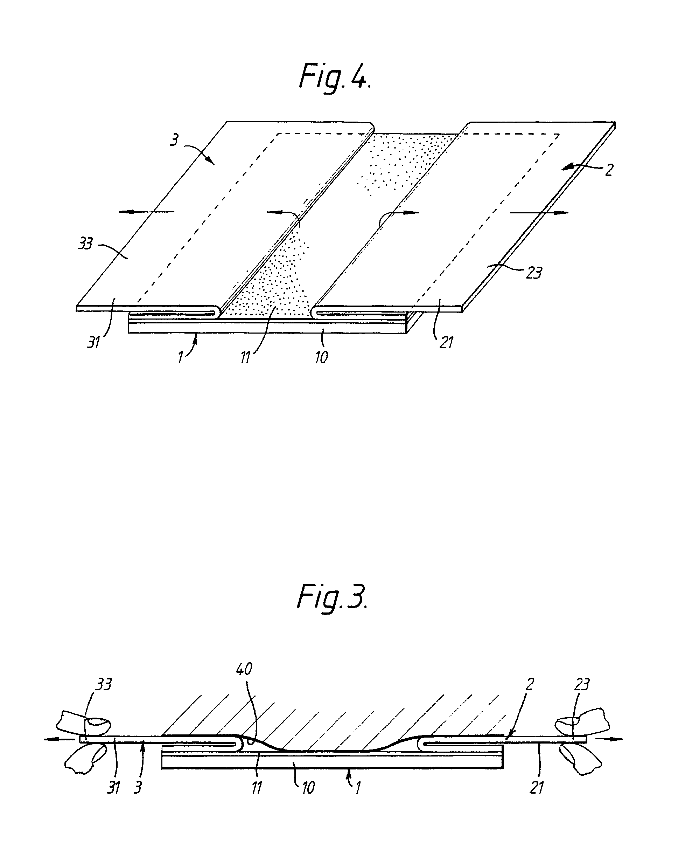

The assembly of dressing and release sheets is stored in this configuration and is presented up to the skin 40 of the user in the manner shown in Figure 2. Both release sheets 2 and 3 remain in place so that the adhesive does not come into contact with the finger of the person applying the dressing. The assembly is held loosely against the skin 40 so that the exposed, overlying parts 21 and 31 of the release sheets 2 and 3 touch the skin. The free ends 23 and 33 of the release sheets 2 and 3 are then gripped and pulled outwardly, parallel to the dressing 1 and the skin 40, away from one another, as shown in Figure 3. As this is done, the release sheets 2 and 3 peel away from their folded edges 22 and 32 from the centre of the dressing. When a central region of the adhesive surface is exposed between the release sheets 2 and 3, this is pressed against the skin 40 and the remaining part of the release sheets are pulled away. Alternatively, the two release sheets could be removed one at a time, the exposed part of the dressing being pressed against the skin prior to removal of the other release sheet. In either case, the outward pull exerted by the release sheets holds the dressing 1 in tension and prevents the formation of wrinkles. Because the adhesive is not handled during application it does not become contaminated and does not lose any of its adhesive qualities.

It will be appreciated that the invention could be used with other adhesives dressing including drapes and bandages and is not confined to those which are made of a thin plastics material.

## Claims

1. An adhesive dressing assembly comprising a flexible dressing with an adhesive surface for applying to a patient and two release sheets, characterised in that each release sheets (2 and 3) is folded substantially in half, that each sheet has a first half (20, 30) releasably adhered to a respective different part of the dressing (1) and a second half (21, 31) folded back to overlay the first half, that the folded edges (22, 32) of the two release sheets lie adjacent one another, and that the second half (21, 31) of each sheet (2, 3) extends to respective opposite edges of the assembly so that the release sheets can be removed from the dressing when the assembly is held up to the skin (40) by pulling the respective second halves outwardly from opposite edges of the assembly substantially parallel to the dressing (1) so that the release sheets (2, 3) peel away from the adhesive surface (11) from the centre of the dressing.

2. An adhesive dressing assembly according to Claim 1, characterised in that the folded edge (32) of one release sheet (3) overlaps the folded edge (22) of the other release sheet (2).

3. An adhesive dressing assembly according to Claim 1 or 2, characterised in that the second half (21, 31) of each release sheet (2, 3) has a free end (23, 33) that projects beyond the edge of the dressing (1).

4. An adhesive dressing assembly according to any one of the preceding claims, characterised in that the release sheets (2, 3) are less flexible than the dressing (1), and that the release sheets (2, 3) maintain the dressing (1) in a generally flat configuration and control wrinkling during handling and application of the dressing to the patient whereby the dressing (1) resumes its flexible nature after removal of the release sheets (2, 3) and application to the patient so as not to inferfere with the flexible functioning of the dressing on the patient's skin (40).

5. An adhesive dressing assembly according to any one of the preceding Claims, characterised in that the dressing is less than 300 micron thick.

6. An adhesive dressing assembly according to Claim 5, characterised in that the dressing is less than 100 micron thick.

7. An adhesive dressing assembly according to any one of the preceding claims, characterised in that the dressing (1) comprises a continuous backing layer (10) of an unreinforced polyurethane film and an adhesive surface (11) of a continuous pressure-sensitive adhesive, that both the backing layer (10) and the adhesive (11) are permeable to moisture vapour, and that at least the backing layer (10) is non-permeable to liquid water.

8. An adhesive dressing assembly according to any one of the preceding claims, characterised in that the release sheets (2, 3) are of siliconized paper.

# Fig. 1.

# Fig. 2.

5

EP 0 434 258 A2



## Fig. 4.

## Fig. 3.